## Europäisches Patentamt
## European Patent Office
## Office européen des brevets

(19)

(11) Publication number: **0 059 535**
**A1**

(12)

# EUROPEAN PATENT APPLICATION

(21) Application number: **82300576.4**

(22) Date of filing: **04.02.82**

(51) Int. Cl.³: **A 23 L 1/34**
**A 61 K 9/20, A 23 L 1/10**

(30) Priority: **06.02.81 GB 8103651**

(43) Date of publication of application:
**08.09.82 Bulletin 82/36**

(84) Designated Contracting States:
**AT BE CH DE FR GB IT LI LU NL SE**

(71) Applicant: **MENLEY & JAMES LABORATORIES, LIMITED**
**1 Franklin Plaza**
**Philadelphia Pennsylvania 19101(US)**

(72) Inventor: **Engelbert, Herbert**
**7302 Sharpless Road**
**Melrose Park Pennsylvania 19126(US)**

(74) Representative: **Johnston, Walter Paul**
**Patent Department SMITH KLINE & FRENCH**
**LABORATORIES LTD Mundells**
**Welwyn Garden City Hertfordshire AL7 1EY(GB)**

(54) Dietary fibre-containing compositions.

(57) The invention provides a chewable table comprising from 17 to 95% by weight of dietary fibre and a binding agent. The tablet is made by a process which comprises intimately mixing, in the required proportions, a dietary fibre and a binding agent so as to obtain a cohesive mixture and forming the mixture into a tablet.

EP 0 059 535 A1

TITLE MODIFIED
see front page

## Compositions

This invention relates to dietary fibre-containing tablets and a process for their preparation.

Dietary fibre is that part of plant material which is resistant to digestion in the human gastro-intestinal tract. (H.C.Trowell Atherosclerosis 1972 16 138-40). It is being realised increasingly that dietary fibre has an important role to play in many aspects of nutrition. One particular aspect is weight control. It was been established recently that weight loss by obese patients receiving a calorie controlled diet can be increased when part of the diet consists of a high bran food stuff. Bran is common source of dietary fibre.

Bran based weight control products generally consist of a tablet of compressed raw bran. One particularly successful product is Bran-Slim (Bran-Slim is a Registered Trade Mark). Products of this type suffer from the limitation that they can only contain restricted amounts of fibre because raw bran is relatively poorly compressable.

We have now found that it is possible to formulate fibre-containing tablets in such a way that they contain more fibre, than was possible with previously known formulations.

Accordingly the present invention provides a chewable tablet comprising from 17 to 95% by weight of dietary fibre and a binding agent.

Preferably the fibre is one that expands on ingestion. Preferably the fibre is water-absorbent. In particular it is a partially dried fibre. Preferably cooked bran is present as the fibre.   In particular the cooked bran is extruded cooked bran.   In practice the tablet can contain from 40 to 95% of cooked bran. Generally the tablet contains at least 50% but not more than 80% of cooked bran.

Especially palatable tablets are obtained when the amount of cooked bran is 70% or below.   In particular the tablet can contain 57% by weight of cooked bran.

Raw bran can be obtained from any grain hull e.g. wheat, corn, maize and oats.   It will be appreciated that the cooking of bran is not an exact science but cooked bran can be recognised by the food technologist.   Preferably the cooked bran in this invention is cooked wheat bran. The binding agent can by any such agent commonly employed in the food or pharmaceutical arts.   Examples of these agents from pharmaceutical art are polyvinylpyrrolidone, polyethylene glycol, starch paste and gelatin.   Examples of food ingredients which can function as binding agents in the tablets of this invention are raisin and fructose. The term raisin used herein means raisin pulp or puree.

Preferably the binding agent is raisin or fructose because besides acting as binding agents, they impart a pleasant flavour to the tablets.   Raisin and fructose can be used separately or together.   Preferably the tablet contains up to 35% by weight of raisin.   In particular the tablet can contain 25% by weight of raisin.   When raisin is present in small amounts e.g. for flavouring purposes only then in practice the minimum amount will be 10%.   The tablet can contain up to 15% (but preferably 10%) by weight of fructose.   When fructose is present in small amounts (e.g. for flavouring purposes only) in practice the minumum amount will be 0.5%.   Brown sugar can also be present in an amount up to 5% by weight.   In practice the minimum amount of brown sugar will be 0.5%.

The tablets of this invention can be fortified with nutritional supplements for example vitamins (in particular Vitamins A, $B_1$, $B_2$, $B_6$, and C), pro-vitamins (e.g. ß-carateen), assimilable calcium and iron.

The tablets can also contain a flavouring agent, a lubricant to assist tableting and a preservative for example calcium propionate.

The tablet of the invention can be made in unit dose size or it can be made in a multi-dose size which is marked in such a way as the consumer can cut or break off individual unit doses.

A unit dose consists of a 4.5 g tablet containing 2.1/g of cooked bran. One or two such tablets can be taken before meals.

The tablets of this invention can be prepared by a process which comprises intimately mixing, in the required proportions, a binding agent and a dietary fibre so as to obtain a cohesive mixture and forming the mixture into a tablet.

The step of intimately mixing the dietary fibre and binding agent is preferably carried out in a high shear mixer. The tablets can be formed by compression. This compression step can be carried out by conventional techniques and the material will be compressed at a pressure which gives satisfactory cohesion of the ingredients.

Where cooked bran is present as the fibre, the tablets of this invention can be prepared by cooking raw bran and adjusting the moisture content, if required, to form a cohesive bran before mixing with the binding agent.

Raw bran can be cooked in a number of ways for example by boiling in water or by extrusion cooking. The technique of extrusion cooking for bran is known from the cereal art and extrusion cooking in the process of this invention can be carried out in the same general way. The bran is cooked sufficiently to allow it to be formed into the tablets of the invention.

The raw bran (which typically contains 25-30% by weight of moisture) is conveyed into an extrusion cooker with pre-conditioning water. Typically bran can be conveyed into the cooker at from 400 to 635 kg $min^{-1}$ wet weight with pre-conditioning water at from 1.75 to 2.1 kg $min^{-1}$. Typically the extrusion cooker has a 12-hole (0.6 cm diameter) end die-plate outlet nozzle having a cutting knife. The nozzle is maintained at a temperature of 140-155°C during the extrusion. The nozzle operates at an outlet pressure of 20-35 atmos. The bran pre-conditioning water mix has a residence time of from 30 to 40 sec in the nozzle.

Emergent extrudate can be sliced into small lengths (approx 2.5 cm) and conveyed to a continuous drying oven where it is dried to a moisture content of 10% or less and preferably substantially 4% to 6% by passing the material through the oven which is at a temperature of from 70 to 115°C (preferably 110°C) with a residue time of from 25 to 35 min.

The dried cooked bran can be milled in a high shear cutter mixer, screened to a particle size range between 6 and 30 U.S standard mesh, blended with raisin and thereafter with brown sugar. Before forming this mixture into tablets, fructose, nutritional supplements, lubricant and preservative can be added.

The following Examples illustrate the invention.

## EXAMPLES

### Example 1

Raw wheat table bran (88,000 kg; wet weight containing 28% moisture) was passed at a rate of 600 kg $min^{-1}$ with pre-conditioning water at a rate of 1.9 kg $min^{-1}$ through a 100 horse power screw-feed extrusion cooker fitted with a 12-hole (0.6 cm diameter) end die plate outlet nozzle having a knife. The nozzle was initially heated to 140°C with steam and maintained at a temperature in the range 140-155°C throughout the extrusion step. The outlet pressure of the extrusion nozzle was 25 atmos. The bran/pre-conditioning water mix has a residence time of approx. 33 sec in the nozzle. Emerging extrudate was chopped into approx. 2.5 cm lengths with the knife.

The chopped extruded bran was conveyed by a belt through a continuous drying oven having a path length of approx. 13 m. The bran was dried to a water content of approx. 4% by passing the material through the oven at a temperature of 100°C so that it has a residence time in the oven of 28 min.

The dried material was milled using a high shear cutter (Berkel Inc Model VCM-40 vertical cutter mixer) and screened to a particle size of 6-30 U.S. standard mesh.

A portion of milled bran (60.0 kg) was mixed with whole raisins (21.8 kg) and the mixture blended in a high shear cutter-mixer until a bran/raisin homogenate was obtained (the initial mix). The initial mix was mixed, maintaining the temperature in the range 70-90°C, with brown sugar (18.2 kg) in a high shear cutter-mixer until a homogenate (the second mix) was obtained. The second mix was spread out on trays in an air-conditioned atmosphere(35-45% relative humidity) and allowed to cool until it reached ambient temperature.  Any caking was broken up by passing the cooled second mix through a Stokes Oscillating Granulator low shear sizing machine and sieving through a No. 8  U.S. standard mesh screen producing a granular product (the base granulation).

To a mixture of vitamin blend (MJ1, 91.8 g), flavour, (hazelnut H+R 60580; 15.0 g) calcium proprionate (17.0 g) fructose (89.9 g) and lubritab (11.3 g) was added an equal weight (225.0 g) of base granulation.  These ingredients were mixed together and more base granulation (4,050 g) was added so that a total of 4,275.0 g base granulation was present.  This mixture was then compressed into rectangular tablets 19.844 mm wide by 27.781 mm long by 6.35 mm thick each weighing 4.500 g $^+_-$ 0.045 g.

Example 2

To a mixture of vitamin blend (MJ1, 91.8 g) flavour (vanilla H&R 38857; 4.5 g) calcium propionate (17.0 g), fructose (101.4 g) and lubritab (11.3 g) was added an equal weight (226.0 g) of base granulation.  These ingredients were mixed together and more base granulation (4,049.0 g) was added until a total of 4,275.0 g of base granulation was present.   This mixture was compressed in to rectangular tablets of a size and weight described in Example 1.

Example 3

To a mixture of vitamin blend (MJ1 91.8 g) calcium propionate (17.0 g), fructose (104.9 g) and lubritab (11.3 g) was added an equal weight (225.0 g) of base granulation.   These ingredients were mixed and more base granulation (4,050.0 g) was added until a total of 4,275.0g was present.    This mixture was compressed into rectangular tablets of a size and weight described in Example 1.

The flavours H+R 38857 and H+R 60580 are supplied by Haarman and Reimer Inc. New Jersey.

Vitamin blend MJl contains dicalcium phosphate (49 parts by weight) and ferrous sulphate (1 part by weight). Extrusion cooked bran according to these Examples contains 42-45% of dietary fibre.

0059535

14077                                          - 8 -

Claims :-                                                    <u>EPO</u>

1. A chewable tablet comprising from 17 to 95% by weight of dietary fibre and a binding agent.

2. A tablet as claimed in claim 1 where the fibre is one that expands on ingestion.

3. A tablet as claimed in claim 1 or claim 2 where the fibre is water-absorbent.

4. A tablet as claimed in claim 3 where the fibre is a partially dried fibre.

5. A tablet as claimed in any one of claims 1 to 4 where cooked bran is present as the fibre, the amount of cooked bran being 40-95% by weight.

6. A tablet as claimed in claim 5 where the bran is extrusion cooked bran.

7. A tablet as claimed in claim 6 containing 50-80% of extrusion cooked bran.

8. A tablet as claimed in claim 7 containing substantially 57% of extrusion cooked bran.

9. A tablet as claimed in any one of claims 1 to 8, and containing raisin, the amount of raisin being up to 35% by weight.

10. A tablet as claimed in any one of claims 1 to 9, and containing fructose, the amount of fructose being up to 15% by weight.

11. A tablet as claimed in any one of claims 1 to 10 and containing brown sugar the amount of brown sugar being up to 5% by weight.

12. A process for preparing a tablet as claimed in any one of claims 1 to 11, which comprises intimately mixing, in the required proportions, a dietary fibre and a binding agent so as to obtain a cohesive mixture and forming the mixture into a tablet.

European Patent Office

**EUROPEAN SEARCH REPORT**

EP 82 30 0576

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | GB-A-1 507 867 (NORGINE)<br>*Claims 1,6,9,10,11; page 2, lines 9-16* | 1-5,12 | A 23 L 1/34<br>A 61 K 9/20<br>A 23 L 1/10 |
| | --- | | |
| X | GB-A-1 571 251 (M.HANSSEN)<br><br>*Claims 1,2; page 2, column 1, lines 8-36* | 1-4,9, 10,12 | |
| | --- | | |
| X,Y | GB-A-2 000 426 (UNITED STATES OF AMERICA)<br>*Claims 1,6,8,10,12,16,27; page 4, lines 26-30; examples 1-12* | 1-8,11 ,12 | |
| | --- | | |
| Y | GB-A-1 561 190 (WEETABIX)<br>*Claims 1,2,3,11-14; page 4, lines 42-56; examples 1 to 4* | 6-8 | |
| | ----- | | |

**TECHNICAL FIELDS SEARCHED (Int. Cl. ³)**

A 23 L 1/00
A 23 P 1/00
A 61 K 9/00

The present search report has been drawn up for all claims

| Place of search | Date of completion of the search | Examiner |
|---|---|---|
| THE HAGUE | 12-05-1982 | DESMEDT G.R.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82